# EUROPEAN PATENT APPLICATION

(11) **EP 4 660 296 A1**
(43) Date of publication of application: **10.12.2025**
(21) Application number: 24911180.8
(22) Date of filing: 25.12.2024
(51) Int. Cl.: C12N 1/21, C07K 14/245, C12N 9/48, C12N 15/31, C12P 13/22

(54) **ENGINEERED BACTERIUM, CONSTRUCTION METHOD THEREFOR AND USE THEREOF**

(30) Priority: 26.12.2023 CN 202311807065
(71) Applicant: Innobio Corporation Limited, Dalian, Liaoning 116600 (CN); Innobio (Hei Long Jiang) Limited, Jiamusi, Heilongjiang 156100 (CN)
(72) Inventor: FAN, Chao, Dalian, Liaoning 116600 (CN); QI, Jiakun, Dalian, Liaoning 116600 (CN); HONG, Hao, Dalian, Liaoning 116600 (CN); LIU, Jun, Dalian, Liaoning 116600 (CN); CHEN, Jianbin, Dalian, Liaoning 116600 (CN); WU, Wenzhong, Dalian, Liaoning 116600 (CN)
(74) Representative: Dai, Simin
(86) International application number: PCT/CN2024/142151
(87) International publication number: WO 2025/140256

(57) **Abstract**

An engineered strain for improving tryptophan production and a construction method and use thereof are provided. By screening out a strain capable of tolerating high-concentration tryptophan and performing genomic sequencing and protein sequence analysis on the strain, it is found that certain proteins in the strain undergo point mutations and these mutations are capable of enhancing tryptophan production. To increase tryptophan production, protein sequences encoded by fadR or pepD genes in a parent strain are modified. These modifications result in an engineered strain with significantly higher tryptophan production compared to the parent strain. Under scaled-up production conditions, the tryptophan production reaches 62.38 ± 5.80 g/L in a 5 L fermenter, with a glucose-to-tryptophan yield of 24.1%. Compared to an original strain, tryptophan production increases by 1.48-fold, and the glucose-to-tryptophan yield improves by 1.26-fold. The biological materials and its use belong to the technical field of molecular biology and possess broad practical application value.

## Description

### CROSS REFERENCE TO RELATED APPLICATIONS

This application claims priority to Chinese Patent Application No. 202311807065.2, filed on December 26, 2023, the entire contents of which are incorporated herein by reference.

### TECHNICAL FIELD

The present disclosure relates to the technical field of molecular biology, and in particular, to an engineered strain, a construction method, and a use thereof.

### BACKGROUND

L-tryptophan, as an essential amino acid, is widely used in fields across the food, feed, and pharmaceutical industries. The biosynthesis of tryptophan primarily involves three modules: the central carbon metabolism (CCM) pathway, the shikimate (SHIK) pathway, and the chorismate (CHA) pathway. Due to its long biosynthetic route, requirement for multiple precursors, and strong feedback inhibition, the production efficiency of tryptophan is relatively low, resulting in the production of industrial mature strains struggling to meet market demand. With the continuous advancement of synthetic biology, numerous metabolic engineering strategies have been applied to the construction of cell factories for efficient production of L-tryptophan. Although rational modification of metabolic pathways is goal-oriented and often yields significant results, microorganisms are inherently complex systems. Previous studies showed that the expression of cytidine deaminase only slightly increases the mutation rate and known metabolic networks or engineering strategies cannot achieve the purpose of rapidly enhancing production.

### SUMMARY

To solve the above problem, a strain capable of tolerating high-concentration tryptophan is obtained through tolerance-based screening. Genome sequencing and protein sequence analysis on the strain reveal that certain proteins of the strain undergo point mutations, and these mutants is capable of enhancing tryptophan production. Through inducing mutations in the strain that are unrelated to the main metabolic pathways for target amino acid synthesis and glycolysis, a strain with desirable properties is obtained. However, due to the high degree of complexity of the metabolic network of an organism, the obtained mutation results exhibit significant uncertainty and are difficult to accurately predict and regulate at the early stage of research. Those skilled in the art would not typically consider whether modifications to these proteins could be beneficial for tryptophan production. Inspired by the mutagenesis system based on the fusion of cytidine deaminase and T7 RNA polymerase, it is found that fusing cytidine deaminase with the a subunit of *Escherichia coli* RNA polymerase enables accelerated mutagenesis to levels that support efficient adaptive evolution in *Escherichia coli* without compromising cell viability. Mutagenic evolution is an efficient industrial approach for rapidly enhancing the production capacity of microbial strains. When combined with advanced techniques such as genome sequencing, it enables the integration of productivity improvement with mechanistic investigation, providing a foundation and direction for subsequent rational engineering. At the same time, it boosts the productivity of strain and strengthens international competitiveness.

First aspect of the present disclosure provides an engineered strain for increasing tryptophan production, and the engineered strain is obtained by modification from a parent strain. In some embodiments, the engineered strain comprises a gene encoding at least one of a pepD mutant protein or a fadR mutant protein. A second aspect of the present disclosure provides a method for constructing an engineered strain with increased tryptophan production. In some embodiments, the method comprises obtaining the engineered strain by modifying at least one of a pepD protein or a fadR protein of a parent strain; and under a same culture condition, a tryptophan production of the engineered strain is higher than a tryptophan production of the parent strain.

A third aspect of the present disclosure provides a biomaterial, which is used for increasing tryptophan production.

In some embodiments, the biomaterial comprises a mutant protein, and the mutant protein includes a sequence having at least 80% sequence identity to an amino acid sequence shown in SEQ ID NO: 1 or a sequence having at least 80% sequence identity to an amino acid sequence shown in SEQ ID NO: 2.

In some embodiments, the biomaterial comprises a DNA molecule, and the DNA molecule comprises a gene encoding the mutant protein.

In some embodiments, the biomaterial comprises a gene expression cassette, and the gene expression cassette includes the mutant protein or the gene encoding the mutant protein.

In some embodiments, the biomaterial comprises a recombinant vector, and the recombinant vector includes the mutant protein or the gene encoding the mutant protein.

A fourth aspect of the present disclosure provides a use of an engineered strain in increasing tryptophan production.

In the present disclosure, the production of tryptophan specifically refers to the ability of the strain to produce and accumulate tryptophan in a culture medium.

As used herein, the term "protein" of the present disclosure has the meaning commonly understood by those of ordinary skill in the art.

The "pepD mutant protein" of the present disclosure is obtained by introducing a mutation into an amino acid sequence shown in SEQ ID NO: 4. Specifically, the pepD mutant protein is obtained by substituting amino acid residues at positions 21, 225, and 484 of the amino acid sequence shown in SEQ ID NO: 4 with threonine, alanine, and lysine, respectively. In addition, the scope of the present disclosure also encompasses enzymes derived from *Escherichia coli* that share more than 80%, preferably 90%, more preferably 95%, and most preferably over 99% homology with the amino acid sequence shown in SEQ ID NO: 1 and possess the function of cleaving dipeptides with an unblocked N-terminus. Such enzymes are also within the scope of the present disclosure.

Similarly, the "fadR mutant protein" of the present disclosure is obtained by introducing a mutation into an amino acid sequence shown in SEQ ID NO: 5. Specifically, the fadR mutant protein is obtained by substituting amino acid residues at positions 140 and 171 of the amino acid sequence shown in SEQ ID NO: 5 with threonine and isoleucine, respectively. Additionally, the scope of the present disclosure also encompasses proteins derived from *Escherichia coli* that share more than 80%, preferably more than 90%, more preferably over 95%, and most preferably above 99% homology with the amino acid sequence shown in SEQ ID NO: 2 and possess dual DNA-binding transcriptional regulator activity. Such proteins are also within the scope of the present disclosure.

The term "exogenous" of the present disclosure refers to a system containing a substance that is not originally present. For example, if a gene encoding an enzyme that is not originally present in a given strain is introduced into that strain by means such as transformation, and the enzyme is subsequently expressed in the strain, then the enzyme is considered "exogenous" to that strain.

The term "enhancing" not only refers to an increase in the functional effect beyond the original level due to an increase in the intrinsic activity of the protein itself, but may also be achieved through at least one of the following approaches: increasing the copy number of the nucleotides encoding the protein; modifying the regulatory sequence of the gene encoding the protein; replacing the regulatory sequence of the gene encoding the protein on the chromosome with a strong active sequence; substituting the gene encoding the protein with a mutant gene to enhance the protein's activity; or introducing modifications into the gene encoding the protein on the chromosome to enhance protein activity, and may also non-limitedly include any existing method that can enhance protein activity or enhance activity of the introduced protein compared to its endogenous activity.

The term "activity of introduced protein" has the conventional meaning understood by those skilled in the art, and may be implemented using known methods in the field, including but not limited to: inserting a polynucleotide comprising a polynucleotide sequence encoding the protein into the chromosome; and/or cloning the polynucleotide into a vector and introducing it into a microorganism; and/or increasing the copy number of the polynucleotide directly on the chromosome; and/or modifying the promoter of the polynucleotide encoding the protein to enhance transcription initiation rate; and/or modifying the transcription of the polynucleotide encoding the protein to enhance its activity; and/or altering the translation regulatory sequences of the mRNA carrying the polynucleotide encoding the protein to enhance translation intensity; and/or modifying the polynucleotide itself to improve mRNA stability, protein stability, or relieve feedback inhibition of the protein. It may also non-limitedly include any known method capable of introducing protein activity.

The term "vector" refers to a DNA construct comprising a polynucleotide sequence encoding a target protein, which is operably linked to suitable regulatory sequences to enable the expression of the target protein in a host cell. Once introduced into a suitable host cell, the vector may replicate or function independently of the host genome, or it may be integrated into the host genome. These vectors may not be specifically limited as long as the vector is replicable in the host cell. Examples of vectors include natural or recombinant plasmids, cosmids, viruses, and bacteriophages. For example, pWE15, pET, pUC vectors, or the like. In addition, by inserting the vector into the chromosome of a host cell, the polynucleotide encoding the endogenous target protein on the chromosome may be replaced with a modified polynucleotide. Insertion of a polynucleotide into the chromosome may be carried out using any method known in the art, including but not limited to homologous recombination. The polynucleotide includes DNA and RNA encoding the target protein and may be inserted into the chromosome of the host cell in any form, as long as it can be expressed in the host cell. For example, the polynucleotide may be introduced into the host cell in its native form and/or in the form of an expression cassette. An expression cassette refers to a genetic construct containing all essential elements required for expression, and may also be a self-replicating expression vector. It may include a promoter operably linked to the polynucleotide, transcription termination signals, ribosome binding domains, and translation termination signals.

The term "weakening" of the present disclosure refers to reducing, weakening, diminishing, or eliminating the activity of a protein, such as an enzyme. In specific embodiments, weakening of enzyme activity may be achieved by partially or completely knocking out the gene encoding the enzyme, mutational inactivation or partial inactivation of the gene, altering the gene promoter or translation regulatory region to weaken transcription or translation, modifying the gene sequence to reduce mRNA stability or enzyme structural stability, regulating the gene via sRNA, or combinations of these methods, etc.

The term "host cell" of the present disclosure has the meaning commonly understood by a person of ordinary skill in the art, i.e., a strain that contains a protein or a protein mutant thereof. In other words, the present disclosure may utilize any host cell as long as the cell contains the target protein or a mutant thereof and is capable of producing tryptophan. The host cells may be derived from *Escherichia coli* (*E. Coli*)*.* Specifically, the host of the present disclosure refers to a strain capable of producing tryptophan, i.e., in a culture medium, the bacteria can produce and accumulate tryptophan or secrete tryptophan into the culture medium, thereby obtaining extracellular free tryptophan. In particular, it refers to a strain having the ability to accumulate more tryptophan compared to the wild-type or parent strain. To confer the ability to produce tryptophan to a strain, conventional breeding methods may be employed, such as cultivating auxotrophic mutants, analog-resistant strains, or metabolic control mutants capable of producing tryptophan, as well as cultivating recombinant strains with enhanced activity of enzymes related to amino acid biosynthesis, or combinations of these methods.

The term "a pepD mutant protein and/or a fadR protein mutant" of the present disclosure has a meaning routinely understood by those of skill in the art and may be implemented by methods known in the art, including but not limited to, inserting the polynucleotide comprising polynucleotide sequences encoding the protein into the chromosome, and/or cloning the polynucleotide into a vector and introducing it into microorganisms, and/or directly increasing the copy number of the polynucleotide on the chromosome. It may also non-limitedly include any known methods capable of introducing protein activity.

Those skilled in the art understand that when mutating a wild-type polypeptide to enhance activity, identifying the sites that achieve the desired effect is of greater importance. Based on the teachings of the present disclosure, those skilled in the art would substitute serine at position 21, glycine at position 225, and alanine at position 484 of the amino acid sequence of the pepD protein with threonine, alanine, and lysine, respectively; and substitute alanine at position 140 and leucine at position 171 of the amino acid sequence of the fadR protein with threonine and isoleucine, followed by testing the relevant activities of these mutants.

In addition, it would not be difficult for a person of ordinary skill in the art to know that changing a few amino acid residues in certain regions of a polypeptide, e.g., in non-significant regions, does not essentially alter the biological activity, e.g., sequences obtained by appropriate substitution of certain amino acids do not affect the activity (see, e.g., Watson et al, Molecular Biology of The Gene, Fourth Edition, 1987. The Benjamin/Cummings Pub. Co. P224). As such, a person of ordinary skill in the art would be able to carry out this substitution and ensure that the resulting molecule still has the desired biological activity.

Therefore, it is obvious to obtain further mutants of the pepD and/or fadR proteins of the present disclosure and their mutants, which still retain the corresponding functions and activities, through additional mutagenesis. For example, it is well known to those skilled in the art that adding or deleting several amino acid residues at either terminus of a polypeptide, preferably 1 to 20 residues, more preferably 1 to 15 residues, even more preferably 1 to 10 residues, still more preferably 1 to 3 residues, and most preferably 1 residue, does not affect the function of the resulting mutant. For ease of purification, the skilled persons often attach a 6×His tag to either terminus of the obtained protein, and such a tagged protein retains the same function as the protein without the 6×His tag. The present disclosure should therefore include conserved mutants obtained based on the present disclosure.

Compared with the prior art, the present disclosure has the following beneficial effects.

The present disclosure obtains the engineered strain with an increased tryptophan production compared to the parent strain by modifying a protein sequence expressed by a fadR gene or a protein sequence expressed by a pepD gene in a genome of the parent strain; the parent strain being a tryptophan producing strain; modifying the fadR gene is to weaken its expression level or its protein activity, or even knock out the fadR gene; and modifying the pepD gene is to mutate the pepD gene, enhance its expression level or its protein activity. In the case of large-scale production, the tryptophan production reaches 62.38±5.80 g/L in a 5 L fermenter, with a glucose-to-tryptophan yield of 24.1%. Compared to the original strain, the tryptophan production is increased by 1.48-fold, and the glucose-to-tryptophan yield is increased by 1.26-fold.

The deposit information of the engineered strain of the present disclosure is provided below.

*Escherichia coli* IBEWQ-624 was deposited in the China Center for Type Culture Collection (CCTCC) on August 19, 2024, with a deposit number CCTCC NO: M 20241821, and the deposit address: No. 299, Bayi Road, Wuchang District, Wuhan City, Hubei Province, China.

### BRIEF DESCRIPTION OF THE DRAWINGS

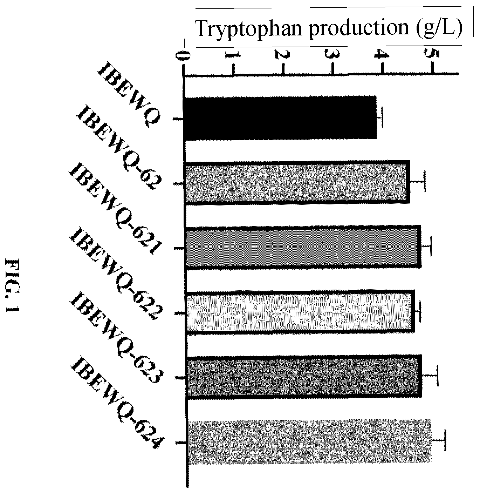

FIG. 1 is a schematic diagram illustrating tryptophan fermentation levels of a strain IBEWQ-62 and strains derived from the strain IBEWQ-62 with different weakening degrees of a fadR gene or/and with different enhancement degrees of a pepD gene in a genome. The diagram indicates the enhanced expression of the pepD gene significantly improves the fermentation performance while weakening or even completely knocking out the fadR gene yields the best improvement in fermentation performance.

### DETAILED DESCRIPTION

One of embodiments of the present disclosure provides an engineered strain, the engineered strain is obtained by modification from a parent strain, and the engineered strain comprises a gene encoding at least one of a pepD mutant protein or a fadR mutant protein.

In some embodiments, the pepD mutant protein includes at least one of mutations S21T, G225A, and A484K. For example, a mutation involved in the pepD mutant protein may be mutations S21T, G225A, or A484K, denoted as pepD^{S21T}, pepD^{G225A}, or pepDA484K, respectively. As another example, the mutation involved in the pepD mutant protein may be the mutations S21T and G225A, mutations G225A and A484K, or mutations S21T and A484K, denoted as pepD^{S21T, G225A}, pepD^{G225A, A484K} or pepD^{A484K, S21}, respectively.

In some embodiments, the mutation involved in the pepD mutant protein may be mutations S21T, G225A, and A484K, denoted as pepD^{A484K, S21, G225A}.

The pepD^{S21T} refers to the serine at position 21 of an amino acid sequence (SEQ ID NO: 4) of the pepD protein being substituted by threonine, and the relevant activity of this protein mutant is then tested.

The pepD^{G225A} refers to the glycine at position 225 of the amino acid sequence of the pepD protein being substituted by alanine, and the relevant activity of this protein mutant is then tested.

The pepD^{A484K} refers to the alanine at position 484 of the amino acid sequence of the pepD protein being substituted by lysine, and the relevant activity of this protein mutant is then tested.

In some embodiments, the pepD mutant protein includes a sequence having at least 80% sequence identity to an amino acid sequence shown in SEQ ID NO: 1. In some embodiments, the pepD mutant protein includes a sequence having at least 85% sequence identity to the amino acid sequence shown in SEQ ID NO: 1. In some embodiments, the pepD mutant protein includes a sequence having at least 90% sequence identity to the amino acid sequence shown in SEQ ID NO: 1. In some embodiments, the pepD mutant protein includes a sequence having at least 95% sequence identity to the amino acid sequence shown in SEQ ID NO: 1. In some embodiments, the pepD mutant protein includes a sequence having at least 99% sequence identity to the amino acid sequence shown in SEQ ID NO: 1.

In some embodiments, the pepD mutant protein has an amino acid sequence shown in SEQ ID NO: 1.

In some embodiments, the fadR mutant protein includes at least one of mutations A140T and L171I. For example, a mutation involved in the fadR mutant protein may be a mutation A140T or a mutation L171I, denoted as fadR^{A140T} or fadR^{LL71I}, respectively; or the mutation involved in the fadR mutant protein may be mutations A140T and L171I, denoted as fadR^{A140T, L171I}.

The fadR^{A140T} refers to the alanine at position 140 of an amino acid sequence (SEQ ID NO: 5) of the fadR protein being substituted by threonine, and the relevant activity of this protein mutant is tested.

The fadR^{L171I} refers to the leucine at position 171 of the amino acid sequence of the fadR protein being substituted by isoleucine, and the relevant activity of this protein mutant is tested.

In some embodiments, the fadR mutant protein includes a sequence having at least 80% sequence identity to an amino acid sequence shown in SEQ ID NO: 2. In some embodiments, the fadR mutant protein includes a sequence having at least 85% sequence identity to the amino acid sequence shown in SEQ ID NO: 2. In some embodiments, the fadR mutant protein includes a sequence having at least 90% sequence identity to the amino acid sequence shown in SEQ ID NO: 2. In some embodiments, the fadR mutant protein includes a sequence having at least 95% sequence identity to the amino acid sequence shown in SEQ ID NO: 2. In some embodiments, the fadR mutant protein includes a sequence having at least 99% sequence identity to the amino acid sequence shown in SEQ ID NO: 2.

In some embodiments, the engineered strain comprises the pepD mutant protein and the fadR mutant protein. The pepD mutant protein includes mutations S21T, G225A, and A484, and the fadR mutant protein includes mutations A140T and L171I.

In some embodiments, the pepD mutant protein has an amino acid sequence shown in SEQ ID NO: 1, and the fadR mutant protein has an amino acid sequence shown in SEQ ID NO: 2.

The engineered strain in the present disclosure is obtained by modification from the parent strain. The parent strain refers to an original strain used for breeding. For example, the parent strain may originate from a spontaneously mutated strain in production, or from a strain exhibiting traits favorable for further research or application, such as rapid growth or low nutritional requirements. For example, the parent strain may be a strain that has already undergone other mutations or a mutator variant with high sensitivity to mutagens.

In some embodiments, the parent strain is selected from any one of *Escherichia coli, Corynebacterium glutamicum, Bacillus subtilis,* or yeast cells.

In some embodiments, the parent strain is selected from one of a strain IBEWQ, a mutant strain IBEWQ-62, a mutant strain IBEWQ-624, an *Escherichia coli* Nissle1917, *Escherichia coli* BL21, *Escherichia coli* HB101, *Escherichia coli* JM109, *Escherichia coli* DH10B, or *Escherichia coli* MG1655.

In some embodiments, the modification includes enhancing an expression level of a pepD protein of the parent strain.

In some embodiments, the modification includes weakening an expression level of a fadR protein of the parent strain.

In some embodiments, the modification includes knocking out a fadR gene in a genome of the engineered strain including the pepD mutant protein with mutations S21T, G225A, and A484 and the fadR mutant protein with mutations A140T and L171I, and replacing the promoter of a gene encoding the pepD mutant protein in a genome of the parent strain with a strong promoter.

Under a same culture condition, a tryptophan production of the engineered strain is increased compared with the parent strain.

Embodiments of the present disclosure provide a method for constructing an engineered strain. In some embodiments, the method comprises: obtaining the engineered strain by modifying at least one of a pepD protein or a fadR protein of the parent strain.

Under a same culture condition, a tryptophan production of the engineered strain is higher than a tryptophan production of the parent strain.

In some embodiments, the modification includes generating the pepD mutant protein by introducing a mutation at an amino acid site of the pepD protein of the parent strain, wherein the mutation includes at least one of mutations S21T, G225A, and A484K; or overexpressing a gene encoding the pepD protein or a gene encoding the pepD mutant protein using a high-copy plasmid as a vector; or in a genome of the parent strain, replacing the promoter of a gene encoding the pepD protein or the promoter of a gene encoding the pepD mutant protein with a strong promoter; or improving the stability of mRNA transcribed from a gene encoding the pepD protein or a gene encoding the pepD mutant protein.

In some embodiments, the pepD mutant protein includes a sequence having at least 80% sequence identity to the amino acid sequence shown in SEQ ID NO: 1.

In some embodiments, the modification includes: knocking out a gene encoding the fadR protein in the parent strain; or introducing a mutation at an amino acid site of the fadR protein of the parent strain, wherein the mutation includes at least one of mutations A140T and L171I; or in a genome of the parent strain, replacing the promoter of a gene encoding the fadR protein or the promoter of a gene encoding the fadR mutant protein with a weak promoter; or inhibiting translation efficiency or reducing stability of mRNA transcribed from a gene encoding the fadR protein or a gene encoding the fadR mutant protein. In some embodiments, the fadR mutant protein includes a sequence having at least 80% sequence identity to the amino acid sequence shown in SEQ ID NO: 2.

In some embodiments, the modification includes following operations.

The endogenous pepD and/or fadR proteins of the starting strain are directly modified, or the exogenous pepD and/or fadR proteins are modified before being introduced into the starting strain. The modification method is selected from at least one of the following techniques.
a. In the parent strain, the modification of the pepD protein may be any one of the following.
   (a1) Introducing mutations at three amino acid sites of the pepD protein, including mutations S21T, G225A, and A484K, to obtain the pepD mutant protein, referred to as pepD^{S21T, G225A, A484K}, and an amino acid sequence of the pepD mutant protein being shown in SEQ ID NO: 1.
   (a2) Overexpressing a gene encoding the pepD protein or a gene encoding the mutant protein pepD^{S21T, G225A, A484K} using a high-copy plasmid as the vector.
   (a3) In a genome of the parent strain, replacing the promoter of a gene encoding the pepD protein or the promoter of a gene encoding the mutant protein pepD^{S21T, G225A, A484K} with a strong promoter.
   (a4) Improving the stability of mRNA transcribed from a gene encoding the pepD protein or a gene encoding the mutant protein pepD^{S21T, G225A, A484K}
   (a5) Any manner or combination of manners capable of enhancing an expression level of a gene encoding the pepD protein or a gene encoding the mutant protein pepD^{S21T, G225A, A484K}
b. In the parent strain, the modification of the fadR protein may be any one of the following.
   (b1) Knocking out a gene encoding the fadR protein in the parent strain.
   (b2) Introducing mutations at two amino acid sites of the gene encoding the fadR protein, including mutations A140T and L171I, to obtain the fadR mutant protein, referred to as fadR^{A140T, L171I}, and an amino acid sequence of the fadR mutant protein being shown in SEQ ID NO: 2.
   (b3) In a genome of the parent strain, replacing the promoter of a gene encoding the fadR protein or the promoter of a gene encoding the mutant protein fadR^{A140T}, ^{L171I} with a weak promoter.
   (b4) Inhibiting the translation efficiency or reducing the stability of mRNA transcribed from a gene encoding the fadR protein or a gene encoding the mutant protein fadR^{A140T, L171I}.
   (b5) Any manner or combination of manners capable of weakening an expression level of a gene encoding the fadR protein or a gene encoding the mutant protein fadR^{A140T, L1711}.

Further, in specific embodiments of the present disclosure, the parent strain is a tryptophan-producing strain; and the parent strain is selected from any one of *Escherichia coli, Corynebacterium glutamicum, Bacillus subtilis,* yeast cells, or the like. When the parent strain is *Escherichia coli,* it is further preferably selected from one of a strain IBEWQ, a mutant strain IBEWQ-62, a mutant strain IBEWQ-624, an *Escherichia coli* Nissle1917, *Escherichia coli BL21, Escherichia coli* HB101, *Escherichia coli* JM109, *Escherichia coli* DH10B, or *Escherichia coli* MG1655.

In some embodiments, the engineered strain is the mutant strain IBEWQ-624. The mutant strain IBEWQ-624 includes a pepD mutant and a fadR mutant protein, the pepD mutant protein has an amino acid sequence shown in SEQ ID NO: 1, and the fadR mutant protein has an amino acid sequence shown in SEQ ID NO: 2.

In some embodiments, the obtaining the engineered strain includes culturing the parent strain in culture media including different concentrations of tryptophan and measuring a biomass in each culture medium; determining a growth rate of the parent strain based on the biomass; and obtaining the engineered strain capable of increasing a tryptophan production by fermenting a strain with a faster growth rate in a culture medium including high-concentration tryptophan.

In some embodiments, a concentration of the high-concentration tryptophan is in a range of 50 g/L to 70 g/L.

In specific embodiments of the present disclosure, the engineered strain IBEWQ-624 is constructed by knocking out a gene encoding the fadR protein in a genome of the strain IBEWQ, mutating a gene encoding the pepD protein into a gene encoding pepD^{S21T, G225A, A484K} protein, and replacing the promoter of the gene encoding pepD^{S21T, G225A, A484K} protein with a strong promoter PJ23119.

One of embodiments of the present disclosure provides a mutant protein. The mutant protein includes a sequence having at least 80% sequence identity to an amino acid sequence shown in SEQ ID NO: 1 or a sequence having at least 80% sequence identity to an amino acid sequence shown in SEQ ID NO: 2.

In some embodiments, the mutant protein includes a pepD mutant protein, and the pepD mutant protein includes at least one of mutations S21T, G225A, and A484K.

In some embodiments, the mutant protein includes a fadR mutant protein, and the pepD mutant protein includes at least one of mutations A140T and L171I.

One of embodiments of the present disclosure provides a DNA molecule. In some embodiments, the DNA molecule comprises a gene encoding the mutant protein of any one of claims 19-21.

One of embodiments of the present disclosure provides a gene expression cassette. In some embodiments, the gene expression cassette comprises the mutant protein described above or the gene encoding the mutant protein described above.

One of embodiments of the present disclosure provides a recombinant vector. In some embodiments, the recombinant vector comprises the mutant protein described above or the gene encoding the mutant protein described above.

One of embodiments of the present disclosure provides a use of an engineered strain in increasing tryptophan production.

In some embodiments, the use includes fermenting and culturing the engineered strain to obtain tryptophan with increased production.

In some embodiments, the pepD mutant protein included in the engineered strain includes any one of the following: (A1) a protein having an amino acid sequence shown in SEQ ID NO: 1; or (A2) a protein derived from the amino acid sequence shown in SEQ ID NO: 1 containing one or more amino acid substitutions, deletions, and/or insertions, and possessing the same function as the protein having an amino acid sequence shown in SEQ ID NO: 1; or (A3) a protein having an amino acid sequence with at least 99%, 95%, 90%, 85%, or 80% homology to any one of the amino acid sequences defined in (A1) and (A2), and possessing the same function as the proteins defined in (A1) and (A2); or (A4) a fusion protein obtained by linking a tag to the N-terminus and/or C-terminus of any one of the proteins defined in (A1) to (A3).

In some embodiments, the fadR mutant protein included in the engineered strain includes any one of the following: (A1) a protein having an amino acid sequence shown in SEQ ID NO: 2; or (A2) a protein derived from the amino acid sequence shown in SEQ ID NO: 2 containing one or more amino acid substitutions, deletions, and/or insertions, and possessing the same function as the protein having an amino acid sequence shown in SEQ ID NO: 2; or (A3) a protein having an amino acid sequence with at least 99%, 95%, 90%, 85%, or 80% homology to any one of the amino acid sequences defined in (A1) and (A2), and possessing the same function as the proteins defined in (A1) and (A2); or (A4) a fusion protein obtained by linking a tag to the N-terminus and/or C-terminus of any one of the proteins defined in (A1) to (A3).

In some embodiments, the gene encoding the pepD mutant protein includes a DNA molecule having at least 99%, 95%, 90%, 85%, or 80% homology to a DNA sequence defined by a gene encoding the pepD^{S21T, G225A, A484K} mutant protein.

In some embodiments, the gene encoding the fadR mutant protein includes a DNA molecule having at least 99%, 95%, 90%, 85%, or 80% homology to a DNA sequence defined by a gene encoding the fadR^{A140T, L171I} mutant protein.

The present disclosure provides any one of the following biomaterials, which may be used to increase tryptophan production.
(I) Protein: pepD^{S21T, G225A, A484K} mutant protein and/or fadR^{A140T, L171I} mutant protein.
(II) Gene: a gene encoding pepD^{32LT, G225A, A484K} mutant protein and/or a gene encoding fadR^{A140T, L171I} mutant protein.
(III) Expression cassette: an expression cassette including a gene encoding pepD^{S21T, G225A, A484K} mutant protein and/or a gene encoding fadR^{A140T, L171I} mutant protein, or an expression cassette including a DNA fragment of the gene encoding the pepD^{S21T, G225A, A484K} mutant protein and/or a DNA fragment of the gene encoding the fadR^{A140T, L171I} mutant protein.
(IV) Recombinant vector: a recombinant vector including a gene encoding pepD^{S21T, G225A, A484K} mutant protein and/or a gene encoding fadR^{A140T, L171I} mutant protein, or a recombinant vector including a DNA fragment of the gene encoding the pepD^{S21T, G225A, A484K} mutant protein and/or a DNA fragment of the gene encoding the fadR^{A140T, L171I} mutant protein.
(V) Recombinant strain: a recombinant strain including a gene encoding pepD^{S21T, G225A, A484K} mutant protein and/or a gene encoding fadR^{A140T, L171I} mutant protein, or a recombinant strain including a DNA fragment of the gene encoding the pepD^{S21T, G225A, A484K} mutant protein and/or a DNA fragment of the gene encoding the fadR^{A140T, L171I} mutant protein.
   (a) A use of a biomaterial in increasing tryptophan production of a parent strain.
   (b) A use of a biomaterial in producing tryptophan.
   (c) A use of pepD^{S2LT, G225A, A484K} mutant protein or fadR^{A140T, L171I} mutant protein in increasing tryptophan production of a parent strain.
   (d) A use of pepD^{32LT, G225A, A484K} mutant protein or fadR^{A140T, L171I} mutant protein in producing tryptophan.

### Embodiments

The following provides a detailed description of specific embodiments of the present disclosure. However, it should be understood that the scope of protection of the present disclosure is not limited to these specific embodiments.

In the present disclosure, unless otherwise specified, the experimental methods used are conventional methods, and the materials and reagents are commercially available.

### Embodiment 1 Screening for a tolerant strain with accelerated growth rate in tryptophan fermentation broth

Fusion of cytidine deaminase with the a subunit of *Escherichia coli* RNA polymerase can accelerate mutations to levels that support efficient adaptive evolution in *Escherichia coli* without reducing cell viability.

Primers RNAP α-F/R and CDA-F/R were used to amplify a gene sequence encoding the α subunit of *Escherichia coli* RNA polymerase and a gene sequence encoding the cytidine deaminase of a genome of the parent strain, respectively. Then, two end sequences were fused and expressed using the primers RNAP α-F and CDA-R, digested with restriction enzymes EcoRI and NcoI, and then ligated into the temperature-sensitive plasmid pKD46 (GenBank accession no.: MF287367) that has been digested with the same enzymes, to obtain the plasmid denoted as pKAP, and the plasmid pKAP included an arabinose-inducible promoter regulating the expression of the gene of the α subunit of *Escherichia coli* RNA polymerase and the gene of the cytidine deaminase.

The primer sequences used for constructing the plasmid pKAP was as follows:
RNAP α-F: GAATTCatgcagggttctgtgacag; SEQ ID NO: 7.
RNAP α-R: CGATCCGCCACCGCCAGAGCCACCTCCGCCctcgtcagcgatgcttgccggtg; SEQ ID NO: 8.
CDA-F: GGCGGAGGTGGCTCTGGCGGTGGCGGATCGcatccacgttttcaaaccgc; SEQ ID NO: 9.
CDA-R: CCATGGttaagcgagaagcactcgg; SEQ ID NO: 10.

### Embodiment 2

### (1) Construction of a parent strain IBEWQ

The parent strain IBEWQ was derived from *Escherichia coli* W3110 (competent cells of which were commercially available from various biological reagent suppliers). Specifically, at the tnaA locus, the trpE^{S40F, M1293T} DCBA gene was expressed in tandem under the control of the tac promoter, at the trpR locus, AroF^{^P148L, Q152I, N8K}, Aro^{GL76V, P150L, D146N}, tktA, and ppsA genes derived from *Escherichia coli* K-12 were expressed, and at the tyrR locus, the SerA gene derived from *Bacillus subtilis* was expressed. At the same time, the promoters of the tyrA and pheA genes were replaced with the promoter PJ23114.

### (2) Acquisition of a mutant strain IBEWQ-62

The plasmid pKAP was electrotransformed into the strain IBEWQ to generate a strain IBEWQ-pKAP. The strain IBEWQ-pKAP was used as the parent strain and continuously subculture and evolution were performed in seed media (supplemented with ampicillin resistance) including different concentrations of tryptophan. After 12 hours of incubation, each seed medium was diluted twofold, and the biomass in each seed medium was measured individually.

**Table 1 Biomass measurement values under different concentrations of tryptophan**

| Concentration of tryptophan in culture medium (g/L) | Biomass OD₆₀₀ (0 mM L-arabinose) | Biomass OD₆₀₀ (15 mM L-arabinose) |
|---|---|---|
| 30 | 0.69 | 0.67 |
| 40 | 0.56 | 0.55 |
| 50 | 0.48 | 0.48 |
| 60 | 0.27 | 0.36 |
| 70 | 0.10 | 0.30 |

A strain exhibiting accelerated growth rate in a culture medium including high-concentration tryptophan was cultured in a fermentation medium at 37°C to eliminate the plasmid pKAP. After plating and colony purification, a mutant strain with significantly improved tryptophan production was designated as IBEWQ-62.

Composition of a seed medium: 2.4 g/L K₂HPO₄, 9.6 g/L KH₂PO₄, 15 g/L yeast extract, 10 g/L rice bran, 5.0 g/L (NH₄)₂SO₄, 1.0g/L MgSO₄·7H₂O, and 20 g/L glucose, natural pH.

Composition of a shake flask fermentation medium: 20 g/L glucose, 3.0 g/L yeast extract powder, 30 g/L rice bran, 1.6 g/L (NH₄)₂SO₄, 2.0 g/L citric acid, 5.6 g/L K₂HPO₄, 2.0 g/L MgSO₄·7H₂O, 80 mg/L FeSO₄·7H₂O, 4.0 mg/L CoCl₂·6H₂O, 0.6 mg/L CuSO₄·5H₂O, 6.5 mg/L ZnSO₄·7H₂O, 20 mg/L Na₂SO₄, 4.5 mg/L MnSO₄·H₂O, and 20 g/L calcium carbonate, pH=7.2.

### Embodiment 3 Tryptophan produced by fermentation of mutant strains and its genomic sequencing analysis

The parent strain IBEWQ and the mutant strain IBEWQ-62 were subjected to fermentation. First, each strain preserved in glycerol was revived on slant agar, and then transferred into the seed medium to culture IBEWQ and IBEWQ-62 overnight. The obtained seeds of IBEWQ and IBEWQ-62 were inoculated into 500 mL shake flasks containing 100 mL of fermentation medium, followed by cultivation at 220 rpm and 37°C. Samples were collected to measure residual glucose content and tryptophan production. After 45 hours of fermentation, the tryptophan production level of IBEWQ-62 was significantly higher than that of the parent strain.

The genome of the mutant strain IBEWQ-62 was extracted and subjected to whole-genome sequencing. A comparison of genome analysis revealed that point mutations occurred in the IBEWQ-62 genome compared to the parent strain, and the mutations leading to amino acid mutations were summarized in Table 2.

**Table 2 Mutations in the genome of IBEWQ-62 compared to the wild-type strain**

| Gene name | Mutation | Sequence after mutation | Sequence before mutation |
|---|---|---|---|
| Mutated pepD gene | S21T, G225A, and A484K | MSELSQLSPQPLWDIFAKICTIPHP SYHEEQLAEYIVGWAKEKGFHVER DQVGNILIRKPATAGMENRKPVVL QAHLDMVPQKNNDTVHDFTKDPI QPYIDGEWVKARGTTLGADNGIG MASALAVLADENVVHGPLEVLLTM TEEAGMDGAFGLQGNWLQADILI NTDSEEEGEIYMGCAGGIDFTSNL HLDREAVPAGFETFKLTLKGLKGG HSGGEIHVGLaNANKLLVRFLAGH AEELDLRLIDFNGGTLRNAIPREAF ATIAVAADKVDVLKSLVNTYQEILK NELAEKEKNLALLLDSVANDKAALI AKSRDTFIRLLNATPNGVIRNSDV AKGVVETSLNVGVVTMTDNNVEI HCLIRSLIDSGKDYVVSMLDSLGKL AGAKTEAKGAYPGWQPDANSPVM HLVRETYQRLFNKTPNIQIIHAGLE CGLFKKPYPEMDMVSIGPTITGPH SPDEQVHIESVGHYWTLLTELLKEI PKK (SEQ ID NO: 1) | MSELSQLSPQPLWDIFAKICsIPHPSYH EEQLAEYIVGWAKEKGFHVERDQVGNI LIRKPATAGMENRKPVVLQAHLDMVPQ KNNDTVHDFTKDPIQPYIDGEWVKAR GTTLGADNGIGMASALAVLADENVVH GPLEVLLTMTEEAGMDGAFGLQGNWL QADILINTDSEEEGEIYMGCAGGIDFTS NLHLDREAVPAGFETFKLTLKGLKGGH SGGEIHVGLgNANKLLVRFLAGHAEEL DLRLIDFNGGTLRNAIPREAFATIAVAA DKVDVLKSLVNTYQEILKNELAEKEKNL ALLLDSVANDKAALIAKSRDTFIRLLNA TPNGVIRNSDVAKGVVETSLNVGVVTM TDNNVEIHCLIRSLIDSGKDYVVSMLDS LGKLAGAKTEAKGAYPGWQPDANSPV MHLVRETYQRLFNKTPNIQIIHAGLEC GLFKKPYPEMDMVSIGPTITGPHSPDE QVHIESVGHYWTLLTELLKEIPaK (SEQ ID NO: 4) |
| Mutated fadR gene | A140T and L171I | MVIKAQSPAGFAEEYIIESIWNNRF PPGTILPAERELSELIGaTRTTLREV LQRLARDGWLTIQHGKPTKVNNF WETSGLNILETLARLDHESVPQLI DNLLSVRTNISTIFIRTAFRQHPDK AQEVLATANEVADHADtFAELDYN IFRGLAFASGNPIYGLILNGMKGIY TRIGRHYFANPEARSLALGFYHKLS ALCSEGAHDQVYETVRRYGHESG EIWHRMQKNLPGDLAIQGR (SEQ ID NO: 2) | MVIKAQSPAGFAEEYIIESIWNNR FPPGTILPAERELSELIGaTRTTLREVLQ RLARDGWLTIQHGKPTKVNNFWETSG LNILETLARLDHESVPQLIDNLLSVRTNI STIFIRTAFRQHPDKAQEVLATANEVA DHADaFAELDYNIFRGLAFASGNPIYGL ILNGMKGlYTRIGRHYFANPEARSLALG FYHKLSALCSEGAHDQVYETVRRYGHE SGEIWHRMQKNLPGDLAIQGR (SEQ ID NO: 5) |
| Mutated map gene | V94L and Q182N | MAISIKTPEDIEKMRVAGRLAAEVL EMIEPYVKPGVSTGELDRICNDYIV NEQHAVSACLGYHGYPKSVCISIN EVVCHGIPDDAKLLKDGDIINIDVT VIKDGFHGDTSKMFIVGKPTIMGE RLCRITQESLYLALRMVKPGINLRE IGAAIQKFVEAEGFSVVREYCGHGI GRGFHEEPnVLHYDSRETNVVLKP GMTFTIEPMVNAGKKEIRTMKDG WTVKTKDRSLSAQYEHTIVVTDN GCEILTLRKDDTIPAIISHDE (SEQ ID NO: 3) | MAISIKTPEDIEKMRVAGRLAAEVLEMI EPYVKPGVSTGELDRICNDYIVNEQHA VSACLGYHGYPKSVCISINEVVCHGIPD DAKLLKDGDIVNIDVTVIKDGFHGDTS KMFIVGKPTIMGERLCRITQESLYLALR MVKPGINLREIGAAIQKFVEAEGFSVVR EYCGHGIGRGFHEEPQVLHYDSRETNV VLKPGMTFTIEPMVNAGKKEIRTMKDG WTVKTKDRSLSAQYEHTIVVTDNGCEI LTLRKDDTIPAIISHDE (SEQ ID NO: 6) |

The mutation sites of the genes pepD, fadR, and map are as follows.

The mutated pepD gene, denoted as pepD^{S21T,G225A,A484K}, includes three mutations S21T, G225A, and A484K.

The mutated fadR gene, denoted as fadR^{A140T,L171I}, includes two mutations A140T and L171I.

The mutated map gene, denoted as map^{V94L,Q182N}, includes two mutations V94L and Q182N.

### Embodiment 4 Impact of mutated genes on fermentation performance

In an original strain, the promoters of the amino acid sequence mutated genes (fadR, pepD, and map genes) were individually replaced with promoters of different strengths, and the impact of each mutant on valine production in fermentation was compared.

Following standard gene editing procedures, the promoters of the genes pepD, fadR, and map in the strain IBEWQ were individually replaced with a strong promoter PJ23119. Similarly, following standard gene editing procedures, the promoters of the genes pepD, fadR, and map were replaced with a weak promoter PJ23114. The correctly verified strains after construction were activated in the seed medium for 12 to 16 hours and were transferred into the fermentation medium the next day.

After the fermentation was completed, the tryptophan production of each production strain was shown in Table 3.

**Table 3**

| Modified protein | IBEWQ | IBEWQ-62 | PJ23119 | PJ23114 |
|---|---|---|---|---|
| fadR | 3.87±0.11 g/L | 4.53±0.30 g/L | 3.43±0.68 g/L | 4.18±0.15 g/L |
| pepD | | | 4.06±0.69 g/L | 3.68±0.80 g/L |
| map | | | 3.93±0.33 g/L | 3.71±0.06 g/L |

Based on the fermentation results, weakening of the fadR mutant gene significantly contributes to an increase in tryptophan production, whereas strengthening the pepD mutant gene is beneficial to increase tryptophan yield, and the map mutant has no noticeable impact on tryptophan production.

### Embodiment 5: Overexpression of mutated genes or construction of weakened plasmids

In the mutant strain IBEWQ-62, the promoter of the fadR^{A140T,L171I} gene was replaced by PJ23114, denoted as a strain IBEWQ-621.

The promoter of the pepD^{S21T, G225A, A484K} gene was replaced by PJ23119, denoted as a strain IBEWQ-622.

The fadR^{A140T,L171I} gene was knocked out in a genome of the strain IBEWQ-622, denoted as a strain IBEWQ-623.

The tryptophan production results are shown in Table 1. It is evident that the enhanced expression of the pepD gene significantly improves the fermentation performance, while weakening or even completely knocking out the fadR gene yields the best improvement in fermentation performance. Based on this, a combined strain IBEWQ-624 was constructed by knocking out the fadR gene in the genome of the strain IBEWQ-62 and replacing the promoter of the pepD^{S21T,G225A,A484K} gene by PJ23119.

The fermentation level of the strain IBEWQ-624 reaches 4.91 ± 0.28 g/L, with a glucose-to-tryptophan yield of 24.5%. These results demonstrate that weakening the fadR gene is beneficial for improving the fermentation performance, with a complete knockout of the fadR gene representing the most effective strategy.

### Embodiment 6 Scale-up validation

The parent strain IBEWQ and the mutant strain IBEWQ-624 were inoculated into 500 mL shake flasks containing 100 mL of seed medium, respectively, and cultured at 37°C and 200 rpm for 12 to 16 hours, with an OD₆₀₀ values of 11-13. The cultured seed solution was inoculated into a 5 L fermenter at a 10% (v/v) inoculation volume, with an initial aeration rate of 1.5 vvm and an initial agitation speed of 400 rpm. During fermentation, 25% ammonia water was fed into the fermenter to maintain the pH at 7.0. The fermentation temperature was controlled at 37 ± 0.5°C, and the agitation speed and aeration rate were manually adjusted to maintain the dissolved oxygen level in a range of 20% to 30%. After inoculation for approximately 6 hours, a sharp increase in dissolved oxygen level was observed, indicating depletion of the initial glucose. The automatic feeding mode was then activated, and 800 g/L of glucose was fed to maintain the glucose concentration in the fermentation broth below 1 g/L.

After 16 hours of fermentation, samples were taken every 2 to 4 hours for analysis. At 48 hours, the tryptophan production of the parent strain IBEWQ reached 42.12 ± 4.61 g/L, a glucose-to-tryptophan yield reached 19.1%, and the tryptophan production of the mutant strain IBEWQ-624 reached 62.38 ± 5.80 g/L and the glucose-to-tryptophan yield reached 24.1%.

### Embodiment 7 Use of modification manners in other strains

Taking *Escherichia coli* Nissle1917 as a parent strain, in the genome of the parent strain, a fadR gene was knocked out, a pepD gene was mutated to pepD^{S21T,G225A,A484K}, and the promoter of the pepD gene was replaced by PJ23119 to obtain a recombinant strain N-RD. After 40 hours of shake flask fermentation, 0.97 ± 0.06 g/L of tryptophan was measured in the fermentation broth of the parent strain *Escherichia coli* Nissle 1917, while 1.28 ± 0.03 g/L of tryptophan was measured in the fermentation broth of the recombinant strain, representing a 1.32-fold increase in the tryptophan production.

Composition of the shake flask fermentation medium:10 g/L glucose, 5.0 g/L yeast extract powder, 10 g/L rice bran, 6.0 g/L (NH₄)₂SO₄, 3.0 g/L sodium citrate, 2.0 g/L L-glutamine, 1.0 g/L L-serine, 5.6 g/L K₂HPO₄, 3.0 g/L MgSO₄·7H₂O, 65 mg/L FeSO₄·7H₂O, and 20 g/L calcium carbonate, and pH=7.2.

Taking *Escherichia coli* BL21, HB101, JM109, DH10B, and MG1655 as the parent strain, a similar effect of 1.3 to 1.5-fold increase in tryptophan production can also be achieved.

The foregoing description only illustrates preferred embodiments of the present disclosure, but the scope of the present disclosure is not limited thereto. Those skilled in the art may readily conceive of various modifications or substitutions within the technical scope disclosed herein, all of which should be encompassed within the protection scope of the present disclosure. Therefore, the protection scope of the present invention shall be defined by the claims.

## Claims

1. An engineered strain, wherein the engineered strain is obtained by modification from a parent strain, and the engineered strain comprises a gene encoding at least one of a pepD mutant protein or a fadR mutant protein.

2. The engineered strain of claim 1, wherein the pepD mutant protein includes at least one of mutations S21T, G225A, and A484K.

3. The engineered strain of claim 1 or claim 2, wherein the pepD mutant protein includes a sequence having at least 80% sequence identity to an amino acid sequence shown in SEQ ID NO: 1.

4. The engineered strain of claim 1, wherein the fadR mutant protein includes at least one of mutations A140T and L171I.

5. The engineered strain of claim 4, wherein the fadR mutant protein includes a sequence having at least 80% sequence identity to an amino acid sequence shown in SEQ ID NO: 2.

6. The engineered strain of claim 1, wherein the pepD mutant protein has an amino acid sequence shown in SEQ ID NO: 1, and the fadR mutant protein has an amino acid sequence shown in SEQ ID NO: 2.

7. The engineered strain of claim 1, wherein the pepD mutant protein includes mutations S21T, G225A, and A484, and the fadR mutant protein includes mutations A140T and L171I.

8. The engineered strain of claim 1, wherein the modification includes enhancing an expression level of a pepD protein of the parent strain.

9. The engineered strain of claim 1, wherein the modification includes weakening an expression level of a FadR protein of the parent strain.

10. The engineered strain of any one of claims 7-9, wherein the modification includes knocking out a fadR gene in a genome of the engineered strain including the pepD mutant protein with mutations S21T, G225A, and A484 and the fadR mutant protein with mutations A140T and L171I, and replacing a promoter of a gene encoding the pepD mutant protein in a genome of the parent strain with a strong promoter.

11. The engineered strain of any one of claims 1-10, wherein the parent strain is selected from any one of *Escherichia coli, Corynebacterium glutamicum, Bacillus subtilis,* or yeast cells.

12. The engineered strain of any one of claims 1-11, wherein the parent strain is selected from one of a strain IBEWQ, a mutant strain IBEWQ-62, a mutant strain IBEWQ-624, an *Escherichia coli* Nissle1917, *Escherichia coli* BL21, *Escherichia coli* HB101, *Escherichia coliJM109, Escherichia coli* DH10B, or *Escherichia coli* MG1655.

13. The engineered strain of any one of claims 1-12, wherein under a same culture condition, a tryptophan production of the engineered strain is increased compared with the parent strain.

14. A method for constructing an engineered strain with a high tryptophan production, comprising:
obtaining the engineered strain by modifying at least one of a pepD protein or a fadR protein of a parent strain; wherein under a same culture condition, a tryptophan production of the engineered strain is higher than a tryptophan production of the parent strain.

15. The method of claim 14, wherein the modification includes:
generating a pepD mutant protein by introducing a mutation at an amino acid site of the pepD protein of the parent strain, wherein the mutation includes at least one of mutations S21T, G225A, and A484K; or
overexpressing a gene encoding the pepD protein or the pepD mutant protein using a high-copy plasmid as a vector; or
replacing a promoter of a gene encoding the pepD protein or the pepD mutant protein in a genome of the parent strain with a strong promoter; or
improving stability of mRNA transcribed from the gene encoding the pepD protein or the pepD mutant protein.

16. The method of claim 15, wherein the modification includes:
the pepD mutant protein including a sequence having at least 80% sequence identity to an amino acid sequence shown in SEQ ID NO: 1.

17. The method of claim 16, wherein the modification includes:
knocking out a gene encoding the fadR protein of the parent strain; or
generating a fadR mutant protein by introducing a mutation at an amino acid site of the fadR protein of the parent strain, wherein the mutation includes at least one of mutations A140T and L171I; or
replacing a promoter of a gene encoding the FadR protein or the FadR mutant protein in a genome of the parent strain with a weak promoter; or
inhibiting translation efficiency or reducing stability of mRNA transcribed from the gene encoding the FadR protein or the FadR mutant protein.

18. The method of claim 17, wherein the fadR mutant protein includes a sequence having at least 80% sequence identity to an amino acid sequence shown in SEQ ID NO: 2.

19. The method of claim 18, wherein the pepD mutant protein has an amino acid sequence shown in SEQ ID NO: 1, and the fadR mutant protein has an amino acid sequence shown in SEQ ID NO: 2.

20. The method of any one of claims 14-19, wherein the obtaining the engineered strain includes:
culturing the parent strain in culture media including different concentrations of tryptophan and measuring a biomass in each culture medium;
determining a growth rate of the parent strain based on the biomass; and
obtaining the engineered strain capable of increasing a tryptophan production by fermenting a strain with a faster growth rate in a culture medium including high-concentration tryptophan.

21. The method of claim 20, wherein a concentration of the high-concentration tryptophan is in a range of 50 g/L to 70 g/L.

22. A mutant protein, wherein the mutant protein includes a sequence having at least 80% sequence identity to an amino acid sequence shown in SEQ ID NO: 1 or a sequence having at least 80% sequence identity to an amino acid sequence shown in SEQ ID NO: 2.

23. The mutant protein of claim 22, wherein the mutant protein includes a pepD mutant protein, wherein the pepD mutant protein includes at least one of mutations S21T, G225A, and A484K.

24. The mutant protein of claim 23, wherein the mutant protein includes a fadR mutant protein, wherein the pepD mutant protein includes at least one of mutations A140T and L171I.

25. A DNA molecule, comprising a gene encoding the mutant protein of any one of claims 19-21.

26. A gene expression cassette, comprising the mutant protein of any one of claims 19-21 or a gene encoding the mutant protein.

27. A recombinant vector, comprising the mutant protein of any one of claims 19-21 or a gene encoding the mutant protein.

28. A use of the engineered strain of claim 1 in increasing tryptophan production.

29. The use of claim 28, further comprising:
fermenting and culturing the engineered strain to obtain tryptophan with increased production.

30. The use of claim 28, wherein the pepD mutant protein included in the engineered strain includes any one of the following:
(A1) a protein having an amino acid sequence shown in SEQ ID NO: 1; or
(A2) a protein derived from the amino acid sequence shown in SEQ ID NO: 1 containing one or more amino acid substitutions, deletions, and/or insertions, and possessing a same function as the protein having the amino acid sequence shown in SEQ ID NO: 1; or
(A3) a protein having an amino acid sequence with at least 99%, 95%, 90%, 85%, or 80% homology to any one of the amino acid sequences defined in (A1) and (A2), and possessing a same function as the proteins defined in (A1) and (A2); or
(A4) a fusion protein obtained by linking a tag to N-terminus and/or C-terminus of any one of the proteins defined in (A1) to (A3).

31. The use of claim 28, wherein the fadR mutant protein included in the engineered strain includes any one of the following:
(A1) a protein having an amino acid sequence shown in SEQ ID NO: 2; or
(A2) a protein derived from the amino acid sequence shown in SEQ ID NO: 2 containing one or more amino acid substitutions, deletions, and/or insertions, and possessing a same function as the protein having the amino acid sequence shown in SEQ ID NO: 2; or
(A3) a protein having an amino acid sequence with at least 99%, 95%, 90%, 85%, or 80% homology to any one of the amino acid sequences defined in (A1) and (A2), and possessing a same function as the proteins defined in (A1) and (A2); or
(A4) a fusion protein obtained by linking a tag to N-terminus and/or C-terminus of any one of the proteins defined in (A1) to (A3).

32. The use of claim 28, wherein the gene encoding the pepD mutant protein includes a DNA molecule having at least 99%, 95%, 90%, 85%, or 80% homology to a DNA sequence defined by a gene encoding a pepD^{S21T, G225A, A484K} mutant protein.

33. The use of claim 28, wherein the gene encoding the fadR mutant protein includes a DNA molecule having at least 99%, 95%, 90%, 85%, or 80% homology to a DNA sequence defined by a gene encoding a fadR^{A140T, L171I} mutant protein.
